(19) Europäisches Patentamt
European Patent Office
Office européen des brevets

(11) Veröffentlichungsnummer: **0 134 933**
**B1**

(12) # EUROPÄISCHE PATENTSCHRIFT

(45) Veröffentlichungstag der Patentschrift: **07.01.87**

(51) Int. Cl.⁴: **C 07 C 143/77**, C 07 C 143/78, C 07 C 143/74

(21) Anmeldenummer: **84107337.2**

(22) Anmeldetag: **26.06.84**

(54) **2-Ketosulfonamide und Verfahren zu ihrer Herstellung.**

(30) Priorität: **30.06.83 DE 3323511**

(43) Veröffentlichungstag der Anmeldung: **27.03.85 Patentblatt 85/13**

(45) Bekanntmachung des Hinweises auf die Patenterteilung: **07.01.87 Patentblatt 87/2**

(84) Benannte Vertragsstaaten: **BE CH DE FR GB IT LI NL**

(56) Entgegenhaltungen:
**EP - B - 0 001 051**
**DE - A - 3 116 129**

**BULLETIN OF THE CHEMICAL SOCIETY OF JAPAN, Band 52, 1979, Tokio. T. NAGAI et al.: "On the Behavior of Sulfonyl Imide as a Reactive Intermediate. The Reaction with Enamines", Seiten 1102-1106**
**CHEMICAL & PHARMACEUTICAL BULLETIN, Band 21, 1973, Pharmaceutical Society of Japna, Tokyo. HIROAKI YANAGISAWA et al.: "Studies on Chemotherapeutic Agents I", Seiten 1080-1089**
**THE JOURNAL OF THE AMERICAN CHEMICAL SOCIETY, Band 75, 1953, Easton, PA. Mack Printing Company. WILLIAM E. TRUCE et al.: "The preparation of B-Ketosulfonyl Chlorides", Seite 2525**

(73) Patentinhaber: **HOECHST AKTIENGESELLSCHAFT, Postfach 80 03 20, D-6230 Frankfurt am Main 80 (DE)**

(72) Erfinder: **Bender, Albert, Dr., Emil-Nolde-Strasse 11, D-8500 Nürnberg (DE)**
Erfinder: **Günther, Dieter, Dr., Nachtigallenweg 1a, D-6233 Kelkheim (Taunus) (DE)**
Erfinder: **Willms, Lothar, Dr., Grüner Weg 4, D-5463 Unkel (DE)**
Erfinder: **Wingen, Rainer, Dr., Rauenthaler Weg 30, D-6000 Frankfurt am Main 71 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 134 933 B1

**Beschreibung**

2-Ketosulfonamide sind wichtige Zwischenprodukte. Beispielsweise werden sie als Ausgangsprodukte für Pigmente oder Chemotherapeutika genannt.

Zur Herstellung dieser Verbindungen sind bisher zwei Verfahren bekannt geworden.

In dem einen Verfahren dient als Ausgangsprodukt ein 2-Hydroxysulfonamid, das zur Ketoverbindung oxidiert wird. Nach diesem Verfahren können insbesondere aliphatische kurzkettige 2-Ketosulfonamide hergestellt werden. Das Verfahren hat jedoch den Nachteil, dass die zur Oxidation benötigten 2-hydroxysulfonamide nur über schwer herstellbare instabile 2-Hydroxysulfonylchloride zugänglich sind (vgl. Deutsche Offenlegungsschrift 31 16 129).

In dem zweiten Verfahren, nach dem sich insbesondere aromatische 2-Ketosulfonamide herstellen lassen, verwendet man als Ausgangsprodukt ein geeignet substituiertes Acetophenon. Dieses wird mit Schwefeltrioxid in die entsprechende Sulfonsäure überführt. Nach der Umsetzung mit Phosphorchloriden (z.B. Phosphortrichlorid, Phosphorpentachlorid) zum Sulfonsäurechlorid wird dieses mit Aminen zum Sulfonamid umgesetzt (vgl. J. Amer. Chem. Soc. *75*, 2525 (1953)).

Die Verwendung solch reaktiver Verbindungen wie Schwefeltrioxid oder der Phosphorchloride führt jedoch zu Nebenreaktionen, die eine aufwendige Reinigung der Zwischenprodukte erfordern.

Diese Mängel haben zur Folge, dass beide Verfahren zur Herstellung von 2-Ketosulfonamiden nur begrenzt anwendbar sind.

Es wurde nun ein Verfahren gefunden, nach dem sich neue 2-Ketosulfonamide in einfacher Weise dadurch herstellen lassen, dass man bestimmte Enanime mit Sulfamoylhalogeniden umsetzt und das Reaktionsprodukt einer Hydrolyse unterwirft.

Gegenstand der Erfindung sind somit das in den Ansprüchen definierte Verfahren und die nach diesem Verfahren hergestellten 2-Ketosulfonamide.

Die erfindungsgemäss hergestellten 2-Ketosulfonamide sind Verbindungen der Formel I

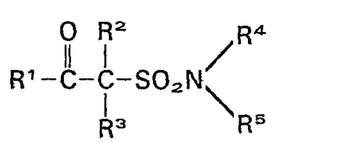

(I),

worin

$R^1$ einen unverzweigten oder verzweigten $C_2$-$C_{10}$-Alkylrest, Aryl-($C_1$-$C_2$)-Alkylrest oder einen Phenylrest bedeutet, der mit einer oder mehreren gleichen oder verschiedenen $C_1$-$C_4$-Alkyl-, $C_1$-$C_4$-Alkoxy-, $C_1$-$C_4$-Alkylthio-, Fluor-, Chlor- oder Brom- oder Phenylresten substituiert sein kann und

$R^2$ Wasserstoff oder einen verzweigten oder unverzweigten $C_1$-$C_{10}$-Alkylrest bedeutet.

$R^1$ und $R^2$ können zusammen mit den beiden benachbarten Kohlenstoffatomen auch einen 3- bis 20-gliedrigen Ring bilden, der an beliebiger Stelle durch einen $C_1$-$C_6$-Alkyl- oder $C_6$-$C_{12}$-Arylrest substituiert sein kann oder an welchen ein Benzorest ankondensiert sein kann, der wiederum an beliebiger Stelle durch ein Halogenatom, einen $C_1$-$C_6$-Alkyl-, $C_1$-$C_4$-Alkoxy- oder $C_1$-$C_4$-Alkylthiorest substituiert sein kann.

$R^3$ bedeutet Wasserstoff oder einen unverzweigten oder verzweigten $C_1$-$C_4$-Alkylrest.

$R^4$ und $R^5$ bedeuten unabhängig voneinander Wasserstoff oder einen $C_1$-$C_6$-Alkyl- oder $C_3$-$C_7$-Cycloalkylrest. Wenn $R^4$ Wasserstoff bedeutet, kann $R^5$ auch einen Phenyl-, Biphenyl- oder Naphthyl-Rest bedeuten, der durch einen oder mehrere gleiche oder verschiedene $C_1$-$C_4$-Alkyl-, $C_1$-$C_4$-Alkoxyreste oder $C_1$-$C_4$-Alkylthioreste oder Halogen (Fluor, Chlor, Brom) substituiert sein kann.

Ausgenommen vom Schutz sollen die Verbindungen der Formel I sein, bei denen

$R^1$ = ein $C_2$-$C_4$-Alkyl- oder Phenylrest ist und $R^2$, $R^3$, $R^4$ und $R^5$ Wasserstoff bedeuten.

$R^1$ = ein Phenylrest ist und $R^2$, $R^3$ und $R^4$ Wasserstoff und $R^5$ einen Phenyl-, Cyclohexyl- oder n-Butylrest bedeuten.

$R^1$ = ein Phenylrest ist und $R^2$ Wasserstoff $R^3$ und $R^4$ einen Methylrest und $R^5$ einen Phenyl-, Cyclohexyl- oder n-Butylrest bedeuten.

$R^1$ = einen Pentylrest ist und $R^2$ und $R^3$ Wasserstoff und $R^4$ und $R^5$ einen Methylrest bedeuten.

Ausgangsstoffe für das erfindungsgemässe Verfahren sind Enamine der Formel II

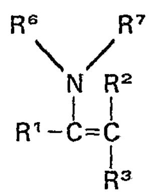

(II),

worin $R^1$, $R^2$ und $R^3$ die vorgenannte Bedeutung haben und $R^6$ und $R^7$ unabhängig voneinander Wasserstoff, einen $C_1$-$C_{20}$-Alkyl-, $C_3$-$C_7$-Cycloalkyl- oder $C_6$-$C_{10}$-Arylrest bedeuten.

$R^6$ und $R^7$ können auch zusammen mit dem benachbarten Stickstoffatom einen gemeinsamen 5-, 6- oder 7-gliedrigen Ring oder zusammen mit dem benachbarten Stickstoffatom und einem Sauerstoff-, Schwefel- oder einem zweiten, gegebenenfalls durch Wasserstoff, einen Alkyl-, Aralkyl- oder Aryl-Rest substituierten Stickstoffatom einen 5-, 6- oder 7-gliedrigen Ring bilden.

Die Enamine II können nach literaturbekannten Verfahren (Enamines-Synthesis, Structure and Reactions, Hrsg. A.G. Cook, M. Dekker Inc., N.Y./London 1969) hergestellt werden.

Bevorzugt werden Verbindungen der Formel II, in denen $R^6$ und $R^7$ zusammen mit dem Stickstoffatom einen Dimethylamino-, einen Diethylamino-, einen Pyrrolidino-, einen Piperidino- oder einen Morpholino-Rest bedeuten.

Die Enamine werden umgesetzt mit Sulfamoylhalogeniden der Formel III

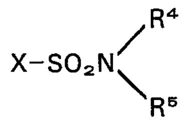 (III),

worin

$R^4$ und $R^5$ die vorgenannte Bedeutung besitzen und X ein Halogenatom bedeutet.

Bevorzugt werden die Sulfamoylchloride (X = Cl) verwendet. Sie können nach literaturbekannten Verfahren hergestellt werden, z.B. durch Umsetzung von Alkylammoniumchloriden mit Sulfurylchlorid (vgl. Liebigs Ann. *729*, 40 (1969)), durch Umsetzung von Alkoholen mit Chlorsulfonylisocyanat (vgl. Deutsche Offenlegungsschrift 24 01 819) oder durch Chlorierung N-substituierter Sulfaminsäurederivate (vgl. J. Org. Chem. *41*, 4 028 (1969)).

Enamin und Sulfamoylhalogenid werden in stöchiometrischer Menge oder mit einem Überschuss des einen oder des anderen Ausgangsstoffes umgesetzt, vorzugsweise in einem unter den Reaktionsbedingungen inerten Lösungsmittel und vorzugsweise in Gegenwart eines Säurefängers.

Die Reaktion wird bei einer Temperatur von −50 bis 65° C, vorzugsweise −30 bis 20° C, durchgeführt.

Als Lösungsmittel werden aliphatische und cycloaliphatische Kohlenwasserstoffe, z.B. Pentan, Hexan, Heptan oder Cyclohexan, aliphatische Halogenkohlenwasserstoffe, z.B. Methylenchlorid, Chloroform, Trichlorethan, aliphatische oder cycloaliphatische Ether, z.B. Diethylether, Diisopropylether, Dioxan oder Tetrahydrofuran, aliphatische Nitrile, z.B. Acetonitril, und aromatische Kohlenwasserstoffe, z.B. Benzol und Toluol, eingesetzt.

Bevorzugt arbeitet man in aliphatischen oder cycloaliphatischen Ethern und besonders bevorzugt in Tetrahydrofuran.

Als Säurefänger kommen organische Verbindungen wie Alkali und Erdalkali-carbonate, -hydrogencarbonate, -oxide und -hydroxide oder organische Basen wie aliphatische oder cycloaliphatische Amine oder Stickstoff-Aromaten in Betracht.

Vorzugsweise werden tertiäre Amine und besonders bevorzugt Triethylamin eingesetzt.

Bevorzugt kann man auch bei einem Verhältnis von 2 bis 2,2 mol Enamin, bezogen auf Sulfamoylhalogenid, das Enamin als Säurefänger einsetzen.

Die Reaktionsmischung wird, gegebenenfalls nach Entfernung des Säurefängers und/oder Wechsel des Lösungsmittels, der Hydrolyse unterworfen, wobei die 2-Ketosulfonamide I in hoher Reinheit aus der Reaktionsmischung abgetrennt werden können.

Die 2-Ketosulfonamide können aus der erhaltenen Reaktionsmischung abgetrennt werden durch Umsetzung der Mischung mit Wasser in Gegenwart katalytischer, stöchiometrischer oder überschüssiger Mengen Säure oder Base oder durch Umsetzung mit wasserhaltigen organischen Lösungsmitteln in Gegenwart katalytischer, stöchiometrischer oder überschüssiger Mengen Säure oder Base oder durch Umsetzung mit wasserhaltigen, sauer oder basisch reagierenden anorganischen Trägermaterialien wie Kieselgur, Kieselgel, Aluminiumoxid, Ionenaustauscher und Harzen mit funktionellen Gruppen. Bevorzugt wird die Umsetzung des Reaktionsgemisches mit überschüssigen Mengen 0,5 bis 5 n Säure, gegebenenfalls in Gegenwart geringer Mengen oben erwähnter Lösungsmittel. Besonders bevorzugt werden 1-2 n Salz- oder Schwefelsäure.

Eine bevorzugte Ausführung des Verfahrens ist, die Reaktionsmischung an saurem oder basischem Trägermaterial chromatographierend zu hydrolysieren und somit, vor allem bei leichter löslichen 2-Ketosulfonamiden, Hydrolyse und Reinigung in einem Schritt vorzunehmen. Besonders bevorzugt wird Kieselgel als Trägermaterial.

Angesichts der komplexen Mischungen und Instabilität der erhaltenen Produkte, die bei der Umsetzung von Enaminen mit Methyl- bzw. Ethylsulfamoylchlorid erhalten wurden (vgl. Bull. Chem. Soc. Jpn. *52* 1 102 (1979)), war es überraschend, dass die aus Enaminen der Formel II und Sulfamylchloriden der Formel III erhaltenen Reaktionsmischungen sich direkt, ohne Isolierung und Reinigung der anzunehmenden Zwischenstufen, in die mit hoher Reinheit anfallenden 2-Ketosulfonamide I überführen lassen. Das erfindungsgemässe Verfahren stellt unter Verwendung gut zugänglicher Ausgangsstoffe und einfacher Reaktionsschritte ohne aufwendige Reinigungsoperationen eine allgemein anwendbare Synthese für 2-Ketosulfonamide dar.

Die erfindungsgemässen Ketosulfonamide der allgemeinen Formel I sind Vorprodukte für neue Pflanzenschutzmittel und Pharmaka. Sie stellen jedoch auch Analoga der Acylessigsäureamide dar, die ihrerseits grosse Bedeutung als Kupplungskomponenten für Azofarbstoffe und -pigmente besitzen.

Die folgenden Beispiele sollen die Erfindung erläutern.

*Beispiel 1:*

*2-Oxo-cyclohexansulfonamid*

Eine Lösung von 16,7 g (0,1 Mol) Morpholinocyclohexen in 200 ml Tetrahydrofuran wurde bei −50° C während 2 h simultan mit Lösungen von 12,7 g (0,11 Mol) Sulfamoylchlorid und 11,1 g (0,11 Mol) Triethylamin in jeweils 100 ml Tetrahydrofuran versetzt, während 2 h von −50 auf 20° C gebracht, filtriert, das Filtrat im Vakuum zur Trockne gebracht und der Rückstand an 400 g Kieselgel mit Methylenchlorid chromatographiert. Die Hauptfraktion ergab nach Umkristallisation aus Isopropanol 9,7 g (54,8% d. Th., bezogen auf Enamin) 2-Oxo-cyclohexansulfonamid vom Schmp. 118-119° C.

*Beispiel 2:*

*2-Oxo-cyclohexylsulfonsäure-cyclohexylamid*

Zur Lösung von 16,7 g (0,1 Mol) Morpholinocyclohexen-(1) und 10,6 (0,105 Mol) Triethylamin in 150 ml Tetrahydrofuran wurden 20,8 g

(0,105 Mol) Cyclohexylaminosulfochlorid in 60 ml Tetrahydrofuran während 2 h bei −50° C getropft, während 4 h auf 20° C gebracht, filtriert, das Filtrat im Vakuum zur Trockne gebracht und über 80 g Kieselgel mit Methylenchlorid chromatographiert. Die Hauptfraktion lieferte nach Kristallisation aus Diisopropylether 14,9 g (57,4%) farblose Kristalle vom Schmp. 105-109° C.

*Beispiel 3:*

*N-Methyl-5-oxo-nonan-4-sulfonamid*

211 g (1 Mol) 5-Morpholino-4-nonen wurden in 1 l Tetrahydrofuran gelöst. In die auf −40° C gekühlte Lösung wurden zuerst 129,5 g (1 Mol) Amidosulfochlorid in 300 ml Tetrahydrofuran und dann noch 101 g (1 Mol) Triethylamin getropft. Man liess auf 20° C erwärmen und filtrierte vom ausgefallenen Triethylammoniumhydrochlorid ab. Dann wurde das Lösungsmittel abdestilliert und der Rückstand eine Stunde mit einem Gemisch aus 1 l Methylenchlorid und 250 ml 2 n Salzsäure verrührt. Die organische Phase wurde abgetrennt, mit Natriumsulfat getrocknet und destilliert. Es wurden 152,3 g (65% d. Th.) eines farblosen Öls vom Kp. 135-138° C bei 0,3 mbar erhalten.

*Beispiel 4:*

*2-(2,4-Dichlorphenyl)-2-oxoethansulfonamid*

128 g (0,5 Mol) 1-Morpholino-1-(2,4-dichlorphenyl)-ethan wurden in 1 l Methylenchlorid gelöst. In die auf −40° C gekühlte Lösung wurden bei dieser Temperatur zuerst 58 g (0,5 Mol) Amidosulfochlorid in 330 ml Methylenchlorid und dann noch 50,5 g (0,5 Mol) Triethylamin getropft. Man liess auf 20° C erwärmen und destillierte das Lösungsmittel ab. Der Rückstand wurde in 500 ml 2 n Salzsäure verrührt. Nach Abfiltrieren wurden 93 g Rohprodukt erhalten, dessen Umkristallisation aus Isopropanol 48,1 g (35% d. Th.) farblose Kristalle vom Schmp. 136-139° C lieferte.

*Beispiel 5:*

*3-Oxo-1,2,3,4-tetrahydro-naphthalin-2-sulfonamid*

Eine Lösung von 19,9 g (0,1 Mol) 2-Pyrrolidino-3,4-dihydronaphthalin in 200 ml Tetrahydrofuran wurde bei −30° C während 1 h mit einer Lösung von 17,3 g (0,15 Mol) Sulfamoylchlorid in 50 ml Tetrahydrofuran sowie anschliessend bei gleicher Temperatur mit einer Lösung von 15,2 g (0,15 Mol) Triethylamin in 50 ml Tetrahydrofuran versetzt, binnen 3 h auf 20° C gebracht, filtriert, das Filtrat i. Vak. vom Lösungsmittel befreit und der Rückstand mit 200 ml 2 n HCl und 40 ml $CH_2Cl_2$ 24 h verrührt. Der erhaltene Feststoff wurde aus Isopropanol umkristallisiert; man erhielt 16,9 g (74,6%) vom Schmp. 145-146° C.

Analog wurden die in der Tabelle 1 aufgeführten Verbindungen erhalten.

*Tabelle 1*

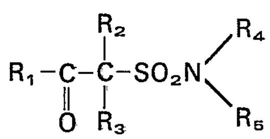

(I)

| Beispiel | $R_1$ | $R_2$ | $R_3$ | $R_4$ | $R_5$ | Schmp. in C° (Kp. in °C/mbar) |
|---|---|---|---|---|---|---|
| 6 | $CH_3-CH_2$ | $CH_3$ | H | H | H | 72-75 |
| 7 | $CH_3-CH_2$ | $CH_3$ | H | H | $CH_3$ | (128/0,2) |
| 8 | $CH_3-(CH_2)_2$ | $CH_3-CH_2$ | H | H | H | 68-71 |
| 9 | $CH_3-(CH_2)_2$ | $CH_3-CH_2$ | H | H | $CH_3$ | (130-135/0,2) |
| 10 | $CH_3-(CH_2)_3$ | $CH_3-(CH_2)_2$ | H | H | H | 69 |
| 11 | $CH_3-(CH_2)_4$ | $CH_3-(CH_2)_3$ | H | H | H | 54 |
| 12 | $(CH_3)_2CH$ | $CH_3$ | $CH_3$ | H | H | 104 |
| 13 | $-(CH_2)_3-$ | | H | H | H | 68 |
| 14 | $-(CH_2)_3-$ | | HJ | H | $CH_3$ | (140-150/0,1) |
| 15 | $-(CH_2)_3-$ | | H | H | n-$C_3H_7$ | (145-150/0,1) |
| 16 | $-(CH_2)_4-$ | | H | H | $CH_3$ | 72 |
| 17 | $-(CH_2)_4-$ | | H | H | $C_2H_5$ | (170-180/0,5) |
| 18 | $-(CH_2)_4-$ | | H | H | n-$C_3H_7$ | (190-200/0,5) |
| 19 | $-(CH_2)_4-$ | | H | H | $C(CH_3)_3$ | 88-90 |
| 20 | $-(CH_2)_4-$ | | H | H | 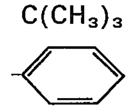 | 199 |
| 21 | $-CH(CH_3)-$ | $-(CH_2)_3-$ | H | H | $CH_3$ | 90 |
| 22 | $-(CH_2)_2-$ | $-CH(CH_3)CH_2-$ | H | H | $CH_3$ | 89 |
| 23 | $-(CH_2)_2-$ | $-CH(Phenyl)CH_2-$ | H | H | H | 132 |
| 24 | $-(CH_2)_2-$ | $-CH(Phenyl)CH_2-$ | H | H | $CH_3$ | 121 |

| Beispiel | $R_1$ | $R_2$ | $R_3$ | $R_4$ | $R_5$ | Schmp. in C° (Kp. in °C/mbar) |
|---|---|---|---|---|---|---|
| 25 | $-(CH_2)_5-$ | | H | H | H | 76 |
| 26 | $-(CH_2)_6-$ | | H | H | H | 109 |
| 27 | $-(CH_2)_{10}-$ | | H | H | $CH_3$ | 135 |
| 28 | 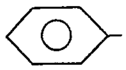 | H | H | H | $CH_3$ | 150 |
| 29 | 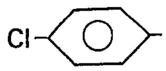 | H | H | H | H | 161 |
| 30 | 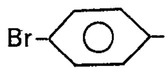 | H | H | H | H | 181 |
| 31 | 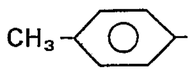 | H | H | H | H | 161 |
| 32 | 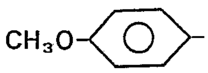 | H | H | H | H | 140 |
| 33 | 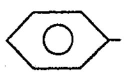 | H | $CH_3$ | H | H | 149 |
| 34 | 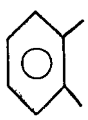 | $-CH_2-$ | H | H | H | 186 |
| 35 | $-CH_2-$ | 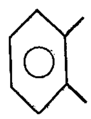 | H | H | H | 167 |
| 36 | 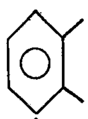 | $-(CH_2)_2-$ | H | H | H | 144 |
| 37 | 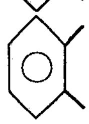 | $-(CH_2)_2-$ | H | H | $CH_3$ | 141 |
| 38 | 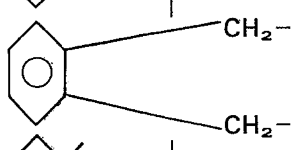 | | H | H | $CH_3$ | 105 |
| 39 | 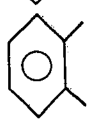 | $-(CH_2)_3-$ | H | H | H | 173 |

*Beispiel 40:*

Dieses Beispiel beschreibt die Herstellung eines Azopigments aus einem erfindungsgemässen 2-Ketosulfonamid und 2-Nitro-4-chloranilin.

3,44 g (20 mMol) 4-Chlor-2-nitroanilin in 10 ml Wasser und 4,2 ml konz. Salzsäure wurden mit 2,8 ml 40%iger Natriumnitritlösung in 5 ml Wasser bei 0-5° C diazotiert. Nach 30 Minuten wurde überschüssiges Nitrit mit Amidosulfonsäure zerstört.

Die so hergestellte Diazoniumlösung liess man bei 0° C und einem pH-Wert von 9-10 in eine Lösung von 4,67 g (20 mMol) 4-Chlor- -sulfonamido-acetophenon in 30 ml Wasser und 15 ml N-Methylpyrrolidon langsam zutropfen. Nach 30 Minuten versetzte man mit 30 ml Eiswasser, saugte den ausgefallenen Farbstoff ab und trocknete. Man erhielt 5 g eines gelben Pigmentes der Formel

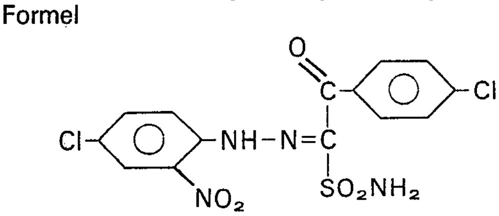

$C_{14}H_{10}Cl_2N_4O_5S$ (417.22)
λ: 422 n m,: 16500 (DMF)

## Patentansprüche

1. Verfahren zur Herstellung von 2-Ketosulfonamiden der Formel I

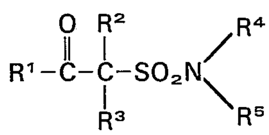

(I),

worin

$R^1$ einen unverzweigten oder verzweigten $C_2$-$C_{10}$-Alkylrest, Aryl-($C_1$-$C_2$)-Alkylrest oder einen gegebenenfalls durch $C_1$-$C_4$-Alkyl-, $C_1$-$C_4$-Alkoxy-, $C_1$-$C_4$-Alkylthio- oder Phenylreste oder Halogenatome substituierten Phenylrest,

$R^2$ Wasserstoff oder einen unverzweigten oder verzweigten $C_1$-$C_{10}$-Alkylrest, wobei

$R^1$ und $R^2$ zusammen mit den beiden benachbarten Kohlenstoffatomen auch einen 3- bis 20-gliedrigen Ring bilden können, welcher an beliebiger Stelle durch einen $C_1$-$C_6$-Alkyl- oder $C_6$-$C_{12}$-Arylrest substituiert sein kann oder an welchen ein Benzorest ankondensiert sein kann, der wiederum an beliebiger Stelle durch ein Halogenatom, einen $C_1$-$C_6$-Alkyl-, $C_1$-$C_4$-Alkoxy- oder $C_1$-$C_4$-Alkylthiorest substituiert sein kann,

$R^3$ Wasserstoff oder einen unverzweigten oder verzweigten $C_1$-$C_4$-Alkylrest und

$R_4$ und $R_5$ unabhängig voneinander Wasserstoff oder einen $C_1$-$C_6$-Alkyl- oder $C_3$-$C_7$-Cycloalkylrest bedeuten, wobei wenn $R^4$ Wasserstoff ist, $R^5$ auch einen gegebenenfalls durch $C_1$-$C_4$-Alkyl-, $C_1$-$C_4$-Alkoxy- oder $C_1$-$C_4$-Alkylthioreste oder Halogenatome substituierten Phenyl-, Biphenyl- oder Naphthylrest bedeuten kann, dadurch gekennzeichnet, dass man ein Enamin der Formel II

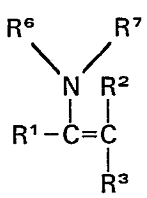

(II),

worin $R^1$, $R^2$ und $R^3$ die vorgenannte Bedeutung haben und $R^6$ und $R^7$ unabhänging voneinander Wasserstoff, einen $C_1$-$C_{20}$-Alkyl-, $C_3$-$C_7$-Cycloalkyl- oder $C_6$-$C_{10}$-Arylrest bedeuten, $R^6$ und $R^7$ auch zusammen mit dem benachbarten Stickstoffatom einen 5-, 6- oder 7-gliedrigen Ring oder zusammen mit dem benachbarten Stickstoffatom und einem Sauerstoff-, Schwefel- oder einem zweiten, gegebenenfalls substituierten Stickstoffatom einen 5-, 6- oder 7-gliedrigen Ring bilden können,

mit einem Sulfamoylhalogenid der Formel III

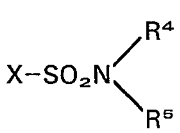

(III),

worin $R^4$ und $R^5$ die obengenannte Bedeutung haben, und X ein Halogenatom bedeutet, bei einer Temperatur von −50 bis 65° C umsetzt und das Reaktionsprodukt einer Hydrolyse unterwirft.

2. Verfahren nach Anspruch 1, dadurch gekennzeichnet, dass man Enamine der Formel II einsetzt, in welcher $R^6$ und $R^7$ zusammen mit dem benachbarten Stickstoffatom einen Dimethylamino-, Diethylamino-, Pyrrolidino-, Piperidino- oder Morpholinorest bedeuten.

3. 2-Ketosulfonamide der Formel I

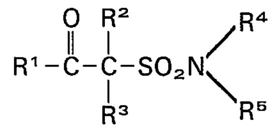

(I),

worin

$R^1$ einen unverzweigten oder verzweigten $C_2$-$C_{10}$-Alkylrest, Aryl-($C_1$-$C_2$)-Alkylrest oder einen gegebenenfalls durch $C_1$-$C_4$-Alkyl-, $C_1$-$C_4$-Alkoxy-, $C_1$-$C_4$-Alkylthio- oder Phenylreste oder Halogenatome substituierten Phenylrest,

$R^2$ Wasserstoff oder einen unverzweigten oder verzweigten $C_1$-$C_{10}$-Alkylrest bedeuten, wobei

$R^1$ und $R^2$ zusammen mit den beiden benachbarten Kohlenstoffatomen auch einen 3- bis 20-gliedrigen Ring bilden können, welcher an beliebiger Stelle durch einen $C_1$-$C_6$-Alkyl- oder $C_6$-$C_{12}$-Arylrest substituiert sein kann oder an welchen ein Benzorest ankondensiert sein kann, der wiederum an beliebiger Stelle durch ein Halogenatom, einen $C_1$-$C_6$-Alkyl-, $C_1$-$C_4$-Alkoxy- oder $C_1$-$C_4$-Alkylthiorest substituiert sein kann,

$R^3$ Wasserstoff oder einen unverzweigten oder verzweigten $C_1$-$C_4$-Alkylrest und

$R_4$ und $R_5$ unabhängig voneinander Wasserstoff oder einen $C_1$-$C_6$-Alkyl- oder $C_3$-$C_7$-Cycloalkylrest bedeuten, wobei wenn $R^4$ Wasserstoff ist, $R^5$ auch einen gegebenenfalls durch $C_1$-$C_4$-Alkyl-, $C_1$-$C_4$-Alkoxy- oder $C_1$-$C_4$-Alkylthioreste oder Halogenatome substituierten Phenyl-, Biphenyl- oder Naphthylrest bedeuten kann,

ausgenommen die Verbindungen der Formel I, in welcher

$R^1$ = ein $C_2$-$C_4$-Alkylrest oder Phenylrest ist und $R^2$, $R^3$, $R^4$ und $R^5$ Wasserstoff bedeuten,

$R^1$ = ein Phenylrest ist und $R^2$, $R^3$ und $R^4$ Wasserstoff und $R^5$ einen Phenyl-, Cyclohexyl- oder n-Butylrest bedeuten,

$R^1$ = ein Phenylrest ist und $R^2$ Wasserstoff, $R^3$ und $R^4$ einen Methylrest und $R^5$ einen Phenyl-, Cyclohexyl- oder n-Butylrest bedeuten,

$R^1$ = ein Pentylrest ist und $R^2$ und $R^3$ Wasserstoff und $R^4$ und $R^5$ einen Methylrest bedeuten.

## Claims

1. A process for the preparation of 2-ketosulfonamides of the formula I

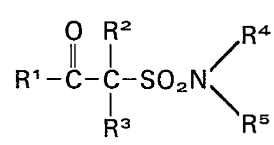

(I),

in which

$R^1$ denotes an unbranched or branched $C_2$-$C_{10}$ alkyl radical, an aryl-($C_1$-$C_2$)-alkyl radical or a phenyl radical optionally substituted by $C_1$-$C_4$ alkyl, $C_1$-$C_4$ alkoxy, $C_1$-$C_4$ alkylthio or phenyl radicals or halogen atoms,

$R^2$ denotes hydrogen or an unbranched or branched $C_1$-$C_{10}$ alkyl radical,

it also being possible for $R^1$ and $R^2$ to form, together with the two adjacent carbon atoms, a 3-membered to 20-membered ring which can be substituted in any position by a $C_1$-$C_6$ alkyl or $C_6$-$C_{12}$ aryl radical or which can be fused with a benzo radical which can in its turn be substituted in any position by a halogen atom, a $C_1$-$C_6$ alkyl, $C_1$-$C_4$ alkoxy or $C_1$-$C_4$ alkylthio radical,

$R^3$ denotes hydrogen or an unbranched or branched $C_1$-$C_4$ alkyl radical and

$R^4$ and $R^5$ independently of one another denote hydrogen or a $C_1$-$C_6$ alkyl or $C_3$-$C_7$ cycloalkyl radical, it also being possible, if $R^4$ is hydrogen, for $R^5$ to denote a phenyl, biphenyl or naphthyl radical optionally substituted by $C_1$-$C_4$ alkyl, $C_1$-$C_4$ alkoxy or $C_1$-$C_4$ alkylthio radicals or halogen atoms, characterized in that an enamine of the formula II

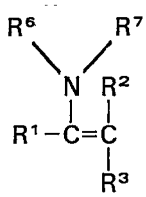

(II),

in which $R^1$, $R^2$ and $R^3$ have the abovementioned meanings and

$R^6$ and $R^7$ independently of one another denote hydrogen or a $C_1$-$C_{20}$ alkyl, $C_3$-$C_7$ cycloalkyl or $C_6$-$C_{10}$ aryl radical, it also being possible for $R^6$ and $R^7$ to form a 5-, 6- or 7-membered ring together with the adjacent nitrogen atom, or a 5-, 6- or 7-membered ring together with the adjacent nitrogen atom and an oxygen atom, a sulfur atom or a second, optionally substituted nitrogen atom,

is reacted with a sulfamoyl halide of the formula III

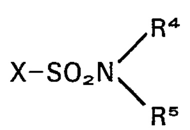

(III),

in which $R^4$ and $R^5$ have the abovementioned meanings and X denotes a halogen atom, at a temperature of −50 to 65° C, and the reaction product is hydrolyzed.

2. The process as claimed in claim 1, characterized in that enamines of the formula II are used in which $R^6$ and $R^7$, together with the adjacent nitrogen atom, denote a dimethylamino, diethylamino, pyrrolidino, piperidino or morpholino radical.

3. A 2-ketosulfonamide of the formula I

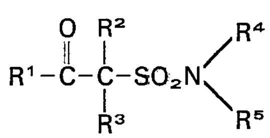

(I)

in which

$R^1$ denotes an unbranched or branched $C_2$-$C_{10}$ alkyl radical, an aryl-($C_1$-$C_2$)-alkyl radical or a phenyl radical optionally substituted by $C_1$-$C_4$ alkyl, $C_1$-$C_4$ alkoxy, $C_1$-$C_4$ alkylthio or phenyl radicals or halogen atoms,

$R^2$ denotes hydrogen or an unbranched or branched $C_1$-$C_{10}$ alkyl radical,

it also being possible for $R^1$ and $R^2$ to form, together with the two adjacent carbon atoms, a 3-membered to 20-membered ring which can be substituted in any position by a $C_1$-$C_6$ alkyl or $C_6$-$C_{12}$ aryl radical or which can be fused with a benzo radical which can in its turn be substituted in any position by a halogen atom, a $C_1$-$C_6$ alkyl, $C_1$-$C_4$ alkoxy or $C_1$-$C_4$ alkylthio radical,

$R^3$ denotes hydrogen or an unbranched or branched $C_1$-$C_4$ alkyl radical and

$R^4$ and $R^5$ independently of one another denote hydrogen or a $C_1$-$C_6$ alkyl or $C_3$-$C_7$ cycloalkyl radical, it also being possible, if $R^4$ is hydrogen, for $R^5$ to denote a phenyl, biphenyl or naphthyl radical optionally substituted by $C_1$-$C_4$ alkyl, $C_1$-$C_4$ alkoxy or $C_1$-$C_4$ alkylthio radicals or halogen atoms, with the exception of the compounds of the formula I in which

$R^1$ is a $C_2$-$C_4$ alkyl radical or phenyl radical and $R^2$, $R^3$, $R^4$ and $R^5$ denote hydrogen,

$R^1$ is a phenyl radical, $R^2$, $R^3$ and $R^4$ denote hydrogen and $R^5$ denotes a phenyl, cyclohexyl or n-butyl radical

$R^1$ is a phenyl radical, $R^2$ denotes hydrogen, $R^3$ and $R^4$ denote a methyl radical and $R^5$ denotes a phenyl, cyclohexyl or n-butyl radical, or

$R^1$ is a pentyl radical, $R^2$ and $R^3$ denote hydrogen and $R^4$ and $R^5$ denote a methyl radical.

**Revendications**

1. Procédé pour préparer des oxo-2 sulfonamides répondant à la formule I:

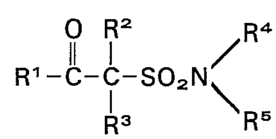

(I)

dans laquelle

$R^1$ représente un radical alkyle en $C_2$-$C_{10}$, ramifié ou non, un radical aryl-alkyle à alkyle en $C_1$ ou $C_2$, ou un radical phényle éventuellement porteur de radicaux alkyles en $C_1$-$C_4$, alcoxy en $C_1$-$C_4$, alkylthio en $C_1$-$C_4$ ou phényles ou d'atomes d'halogènes,

$R^2$ représente l'hydrogène ou un radical alkyle en $C_1$-$C_{10}$, ramifié ou non,

$R^1$ et $R^2$ pouvant également former ensemble, et avec les deux atomes de carbone voisins, un cycle comportant de 3 à 20 maillons, cycle qui peut porter, en un endroit quelconque, un alkyle en $C_1$-$C_6$ ou un aryle en $C_6$-$C_{12}$ et qui peut être condensé à un noyau benzo, celui-ci pouvant à son tour porter, en une position quelconque, un atome d'halo-

gène, un alkyle en $C_1$-$C_6$, un alcoxy en $C_1$-$C_4$ ou un alkylthio en $C_1$-$C_4$,

$R^3$ représente l'hydrogène ou un radical alkyle en $C_1$-$C_4$, ramifié ou non, et

$R^4$ et $R^5$ représentent chacun, indépendamment l'un de l'autre, l'hydrogène, un alkyle en $C_1$-$C_6$ ou un cycloalkyle en $C_3$-$C_7$, et, dans le cas où $R^4$ représente l'hydrogène, $R^5$ peut également représenter un radical phényle, biphénylyle ou naphtyle éventuellement porteur d'alkyles en $C_1$-$C_4$, d'alcoxy en $C_1$-$C_4$, d'alkylthio en $C_1$-$C_4$ ou d'atomes d'halogènes, procédé caractérisé en ce qu'on fait réagir, à une température de −50 à 65° C, une énamine répondant à la formule II

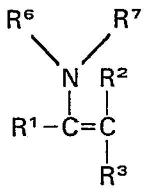

(II),

dans laquelle $R^1$, $R^2$ et $R^3$ ont les significations précédemment données, et $R^6$ et $R^7$ représentent chacun, indépendamment l'un de l'autre, l'hydrogène, un alkyle en $C_1$-$C_{20}$, un cycloalkyle en $C_3$-$C_7$ ou un aryle en $C_6$-$C_{10}$, $R^6$ et $R^7$ pouvant également former ensemble et avec l'atome d'azote voisin un cycle à 5, à 6 ou à 7 maillons, ou ensemble, avec l'atome d'azote voisin et avec un atome d'oxygène ou de soufre ou un deuxième atome d'azote, éventuellement substitué, un cycle à 5, à 6 ou à 7 maillons, avec un halogénure de sulfamoyle répondant à la formule III:

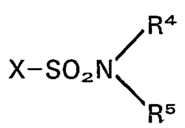

(III)

dans laquelle $R^4$ et $R^5$ ont les significations précédemment données et X représente un atome d'halogène, et on soumet le produit réactionnel à une hydrolyse.

2. Procédé selon la revendication 1, caractérisé en ce qu'on utilise des énamines de formule II dans lesquelles $R^6$ et $R^7$ forment ensemble et avec l'atome d'azote voisin un radical diméthylamino, diéthylamino, pyrrolidino, pipéridino ou morpholino.

3. Oxo-2 sulfonamides répondant à la formule I:

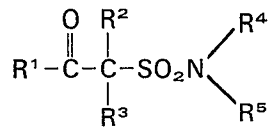

(I)

dans laquelle

$R^1$ représente un radical alkyle en $C_2$-$C_{10}$, ramifié ou non, un radical aryl-alkyle à alkyle en $C_1$ ou $C_2$, ou un radical phényle éventuellement porteur de radicaux alkyles en $C_1$-$C_4$, alcoxy en $C_1$-$C_4$, alkylthio en $C_1$-$C_4$ ou phényles ou d'atomes d'halogènes,

$R^2$ représente l'hydrogène ou un radical alkyle en $C_1$-$C_{10}$, ramifié ou non,

$R^1$ et $R^2$ pouvant également former ensemble, et avec les deux atomes de carbone voisins, un cycle comportant de 3 à 20 maillons, cycle qui peut porter, en un endroit quelconque, un alkyle en $C_1$-$C_6$ ou un aryle en $C_6$-$C_{12}$ et qui peut être condensé à un noyau benzo, celui-ci pouvant à son tour porter, en une position quelconque, un atome d'halogène, un alkyle en $C_1$-$C_6$, un alcoxy en $C_1$-$C_4$ ou un alkylthio en $C_1$-$C_4$,

$R^3$ représente l'hydrogène ou un radical alkyle en $C_1$-$C_4$, ramifié ou non, et

$R^4$ et $R^5$ représentent chacun, indépendamment l'un de l'autre, l'hydrogène, un alkyle en $C_1$-$C_6$ ou un cycloalkyle en $C_3$-$C_7$, et, dans le cas où $R^4$ représente l'hydrogène, $R^5$ peut également représenter un radical phényle, biphénylyle ou naphtyle éventuellement porteur d'alkyles en $C_1$-$C_4$, d'alcoxy en $C_1$-$C_4$, d'alkylthio en $C_1$-$C_4$ ou d'atomes d'halogènes, sauf les composés de formule I dans lesquels:

$R^1$ représente un alkyle en $C_2$-$C_4$ ou un phényle, et $R^2$, $R^3$, $R^4$ et $R^5$ représentent chacun l'hydrogène,

$R^1$ représente un radical phényle, $R^2$, $R^3$ et $R^4$ représentent chacun l'hydrogène et $R^5$ représente un radical phényle, cyclohexyle ou n-butyle,

$R^1$ représente un radical phényle, $R^2$ l'hydrogène, $R^3$ et $R^4$ représentent chacun un radical méthyle et $R^5$ représente un radical phényle, cyclohexyle ou n-butyle,

$R^1$ représente un radical pentyle, $R^2$ et $R^3$ représentent chacun l'hydrogène, et $R^4$ et $R^5$ chacun un radical méthyle.